# EUROPEAN PATENT SPECIFICATION

(11) **EP 2 296 651 B1**
(45) Date of publication and mention of the grant of the patent: **18.07.2012**
(21) Application number: 09742552.4
(22) Date of filing: 08.05.2009
(51) Int. Cl.: C07D 413/14, A61K 31/4375, A61K 31/4709, C07D 471/04

(54) **5-HYDROXYMETHYL-OXAZOLIDIN-2-ONE DERIVATIVES FOR TREATING BACTERIAL INTESTINAL DISEASES**
5-HYDROXYMETHYL-OXAZOLIDIN-2-ON-DERIVATE ZUR BEHANDLUNG BAKTERIELLER DARMERKRANKUNGEN
DÉRIVÉS DE 5-HYDROXYMÉTHYL-OXAZOLIDINE-2-ONE POUR LE TRAITEMENT DE MALADIES INTESTINALES BACTÉRIENNES

(30) Priority: 09.05.2008 WO PCT/IB2008/051854
(43) Date of publication of application: 23.03.2011
(73) Proprietor: Actelion Pharmaceuticals Ltd., 4123 Allschwil (CH)
(72) Inventor: HUBSCHWERLEN, Christian, F-68480 Durmenach (FR); LOCHER, Hans, CH-4102 Binningen (CH)
(74) Representative: Ruhlmann, Eric
(86) International application number: PCT/IB2009/051896
(87) International publication number: WO 2009/136379

(56) References cited:
- WO-A-02/059116
- WO-A-03/032962
- WO-A-2004/096221
- WO-A-2005/058886
- WO-A-2008/056335
- SU-A3- 1 156 597
- US-A- 4 461 773
- ERA-CABRERA L ET AL: "In vitro activities of DA-7157 and DA-7218 against Mycobacterium tuberculosis and Nocardia brasiliensis" ANTIMICROBIAL AGENTS AND CHEMOTHERAPY, AMERICAN SOCIETY FOR MICROBIOLOGY, WASHINGTON, DC, US, vol. 50, no. 9, 1 September 2006 (2006-09-01), pages 3170-3172, XP002473516 ISSN: 0066-4804

## Description

The present invention concerns the use of certain 5-hydroxymethyl-oxazolidin-2-one derivatives for preventing or treating intestinal diseases which are caused by *Clostridium difficile*, *Clostridium perfringens* or *Staphylococcus aureus.*

Certain strains of *Clostridium diffiicile*, *Clostridium perfringens* or *Staphylococcus aureus* are producing toxins which cause diarrhea. The occurrence of these toxin producing strains is often linked with the use of antibiotics.

Among these strains, *Clostridium difficile*, an anaerobic, spore forming, Gram-positive bacterium that produces two enterotoxins (A and B), causes not only diarrhea but also more serious intestinal conditions such as life-threatening pseudomembranous colitis. It is currently the most common source of infectious diarrhea in hospitals and long-term care facilities in the industrialized world. Over the last years, its incidence has progressively increased and is now a significant clinical problem in North America and Europe. The risk factors include prior treatment with antibiotics, age and a compromised immune system resulting from e.g. cytotoxic chemotherapy or organ transplantation.

The current treatment for *Clostridium difficile* associated diarrhea (CDAD) is either vancomycin or metronidazole. In both cases however high relapse rates are observed and the production of toxins and spores is not prevented. Furthermore the treatment with vancomycin or metronidazole is an additional factor to promote the emergence of vancomycin-resistant *Enterococcus spp.* (*E. faecalis* and *E. faecium*) and *Staphylococcus aureus* strains (VRE and VRSA respectively) in the gut (see W.N. Al-Nassir et al., Antimicrob. Agents Chemother., published ahead of print on 28 April 2008). Enterococci are gram-positive cocci that are normal inhabitants of the gastrointestinal tract. However, they can also become significant pathogens, causing endocarditis and urinary tract, bloodstream, and wound infections. Once acquired, intestinal colonization by VRE can last for years , serving as a reservoir for potential infection of the colonized patient and for the spread of VRE to other patients. VRE can appear as co-infection in patients infected with *C. difficile*, or more commonly cause infection in certain high risk patients such as haematology and oncology patients, patients in intensive care units, elderly patients in long-term care facilities and patients who received solid organ transplants.

Therefore there is a need for a better treatment for cases of CDAD and related infections in particular by identifying compounds with strong activity against *C*. *difficile* that significantly reduce the toxin load, the production of spores and which overcome VRE while having a minor impact on the commensal gut flora.

WO 03/032962, WO 2004/096221 and WO 2005/058888 disclose antibacterial compounds containing an oxazolidine moiety and a 1-cyclopropyl-6-fluoro-4-oxo-1,4-dihydroquinoline-3-carboxylic acid or 1-cyclopropyl-6-fluoro-4-oxo-1,4-dihydro-[1,8]naphthyridine-3-carboxylic acid.

Besides, the compounds of formula I as defined hereafter and their antibacterial activity have been previously described in PCT application No. PCT/1B2007/054557.

Various embodiments of the invention are presented hereafter:
i) According to its first main embodiment, the present invention relates to the compounds of formula I 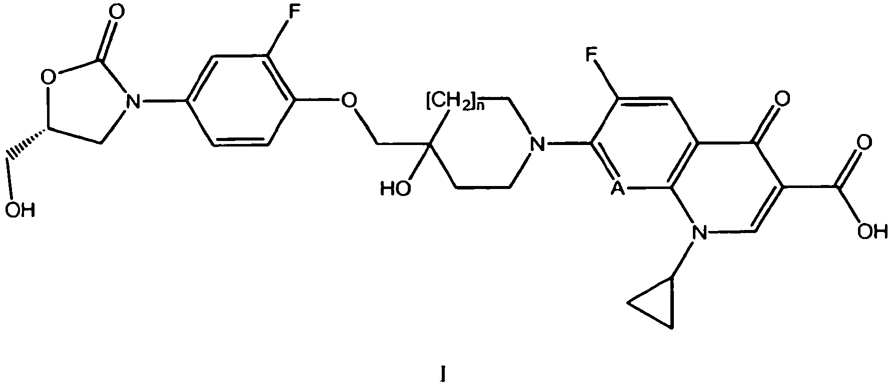 wherein
   A is N or CH; and
   n is 0 or 1;
   or the pharmaceutically acceptable salts thereof, for use in preventing or treating intestinal diseases which are caused by bacteria selected from *Clostridium difficile*, *Clostridium perfringens* or *Staphylococcus aureus*.
   The following paragraphs provide definitions of various terms used herein and are intended to apply uniformly throughout the specification and claims unless an otherwise expressly set out definition provides a broader or narrower definition.
   The term "preventing", "prevent" or "prevention" used with reference to a disease means either that said disease does not occur in the patient or animal, or that, although the animal or patient is affected by the disease, part or all the symptoms of the disease are either reduced or absent.
   The term "treating", "treat" or "treatment" used with reference to a disease means either that said disease is cured in the patient or animal, or that, although the animal or patient remains affected by the disease, part or all the symptoms of the disease are either reduced or eliminated.
   The term "pharmaceutically acceptable salts" refers to non-toxic, inorganic or organic acid and/or base addition salts. Reference can be made to "Salt selection for basic drugs", Int. J. Pharm. (1986), 33, 201-217.
   The term "halogen" refers to fluorine, chlorine, bromine or iodine, and preferably to fluorine or chlorine.
   Unless used regarding temperatures, the term "about" placed before a numerical value "X" refers in the current application to an interval extending from X minus 10% of X to X plus 10% of X, and preferably to an interval extending from X minus 5% of X to X plus 5% of X. In the particular case of temperatures, the term "about" placed before a temperature "Y" refers in the current application to an interval extending from the temperature Y minus 10°C to Y plus 10°C, and preferably to an interval extending from Y minus 5°C to Y plus 5°C; besides, room temperature shall mean in the current patent application 25°C.
ii) Preferably, the compound of formula I as defined in embodiment i), or a pharmaceutically acceptable salt thereof, will allow effective prevention or treatment of diarrhea diseases associated with enterotoxigenic strains of *Clostridium difficile*, *Clostridium perfringens* or *Staphylococcus aureus* without increasing the concentration of vancomycin-resistant enterococci (VRE) in the gut.
iii) More preferably, the compound of formula I as defined in embodiment i), or a pharmaceutically acceptable salt thereof, will allow effective prevention or treatment of diarrhea diseases associated with enterotoxigenic strains of *Clostridium difficile*, *Clostridium perfringens* or *Staphylococcus aureus* and reduction of the concentration of VRE in the gut.
iv) Preferably, the compounds of formula I as defined in one of embodiments i) to iii), or the pharmaceutically acceptable salts thereof, will be such that A is CH.
v) Preferably also, the compounds of formula I as defined in one of embodiments i) to iv), or the pharmaceutically acceptable salts thereof, will be such that n is 1.
vi) According to preferred sub-embodiments of embodiments i) to iii), the compound of formula I or its pharmaceutically acceptable salt will be selected from:
   - 1-cyclopropyl-6-fluoro-7-{4-[2-fluoro-4-((*R*)-5-hydroxymethyl-2-oxo-oxazolidin-3-yl)-phenoxymethyl]-4-hydroxy-piperidin-1-yl}-4-oxo-1,4-dihydro-quinoline-3-carboxylic acid;
   - 1-cyclopropyl-6-fluoro-7-{4-[2-fluoro-4-((*R*)-5-hydroxymethyl-2-oxo-oxazolidin-3-yl)-phenoxymethyl]-4-hydroxy-piperidin-1-yl}-4-oxo-1,4-dihydro-[1,8]naphthyridine-3-carboxylic acid;
   - 1-cyclopropyl-6-fluoro-7-{3-[2-fluoro-4-((*R*)-5-hydroxymethyl-2-oxo-oxazolidin-3-yl)-phenoxymethyl]-3-hydroxy-pyrrolidin-1-yl}-4-oxo-1,4-dihydro-quinoline-3-carboxylic acid;
   and the pharmaceutically acceptable salts thereof.
vii) According to more preferred sub-embodiments of embodiments i) to iii), the compound of formula I or its pharmaceutically acceptable salt will be 1-cyclopropyl-6-fluoro-7-{4-[2-fluoro-4-((*R*)-5-hydroxymethyl-2-oxo-oxazolidin-3-yl)-phenoxymethyl]-4-hydroxy-piperidin-1-yl}-4-oxo-1,4-dihydro-quinoline-3-carboxylic acid or a pharmaceutically acceptable salt thereof.
viii) According to one aspect of embodiments i) to vii), the present invention will relate to the compounds of formula I as defined in one of embodiments i) to vii), or the pharmaceutically acceptable salts thereof, for use in treating the intestinal diseases which are caused by bacteria selected from *Clostridium difficile*, *Clostridium perfringens* and *Staphylococcus aureus.*
ix) According to one preferred aspect of embodiment viii), the present invention will relate to the compounds of formula I as defined in one of embodiments i) to vii), or the pharmaceutically acceptable salts thereof, for use in treating the intestinal diseases which are caused by *Clostridium difficile* (notably by a toxin producing strain of *Clostridium difficile*).
x) According to another main aspect of embodiments i) to vii), the present invention will relate to the compounds of formula I as defined in one of embodiments i) to vii), or the pharmaceutically acceptable salts thereof, for use in preventing the intestinal diseases which are caused by bacteria selected from *Clostridium difficile*, *Clostridium perfringens* and *Staphylococcus aureus.*
xi) According to one preferred aspect of embodiment x), the present invention will relate to the compounds of formula I as defined in one of embodiments i) to vii), or the pharmaceutically acceptable salts thereof, for use in preventing the intestinal diseases which are caused by *Clostridium difficile* (notably by a toxin producing strain of *Clostridium difficile*).
xii) Preferably, the patients in which the intestinal diseases mentioned in embodiments i) to xi) are intended to be prevented will be patients treated with other antibiotics or with antiviral therapies, patients with an immunocompromised system such as cytotoxic chemotherapy or organ transplant patients, elderly (65 years or older) patients, or patients of intensive care units or of long-term care facilities.
xiii) Yet another main embodiment of this invention relates to the use in preventing or treating in a patient an intestinal disease which is caused by bacteria selected from *Clostridium difficile*, *Clostridium perfringens* and *Staphylococcus aureus*, said use comprising the administration to said patient of an effective amount of a compound of formula I as defined in one of embodiments i) to vii), or of a pharmaceutically acceptable salt of such a compound, for a duration sufficient to prevent or treat the intestinal disease.
xiv) Preferably, the use in embodiment xiii) will allow effective prevention or treatment of diarrhea diseases associated with enterotoxigenic strains of *Clostridium difficile*, *Clostridium perfringens* or *Staphylococcus aureus* without increasing the concentration of vancomycin-resistant enterococci (VRE) in the gut.
xv) More preferably, the use in embodiment xiii) will allow effective prevention or treatment of diarrhea diseases associated with enterotoxigenic strains of *Clostridium difficile*, *Clostridium perfringens* or *Staphylococcus aureus* and reduction of the concentration of VRE in the gut.
xvi) According to one aspect of embodiments xiii) to xv), the present invention will relate to a use in treating in a patient an intestinal disease which is caused by bacteria selected from *Clostridium difficile, Clostridium perfringens* and *Staphylococcus aureus,* said use comprising the administration to said patient of an effective amount of a compound of formula I as defined in one of embodiments i) to vii), or of a pharmaceutically acceptable salt of such a compound, for a duration sufficient to treat the intestinal disease.
xvii) According to one preferred aspect of embodiment xvi), the present invention will relate to a use in treating in a patient an intestinal disease which is caused by *Clostridium difficile* (notably by a toxin producing strain of *Clostridium difficile*).
xviii) According to another aspect of embodiments xiii) to xv), the present invention will relate to a use in preventing in a patient an intestinal disease which is caused by bacteria selected from *Clostridium difficile*, *Clostridium perfringens* and *Staphylococcus aureus*, said use comprising the administration to said patient of an effective amount of a compound of formula I as defined in one of embodiments i) to vii), or of a pharmaceutically acceptable salt of such a compound, for a duration sufficient to prevent the intestinal disease.
xix) According to one preferred aspect of embodiment xviii), the present invention will relate to a use in preventing in a patient an intestinal disease which is caused by *Clostridium difficile* (notably by a toxin producing strain of *Clostridium difficile*).
xx) Preferably, the patients subjected to a use in one of embodiments xiii) to xix) will be patients treated with other antibiotics or with antiviral therapies, patients with an immunocompromised system such as cytotoxic chemotherapy or organ transplant patients, elderly (65 years or older) patients, or patients of intensive care units or of long-term care facilities.
xxi) Yet a further aspect of this invention relates to the use of a compound of formula I as defined in one of embodiments i) to vii), or of a pharmaceutically acceptable salt of such a compound, for the manufacture of a medicament intended to prevent or treat an intestinal disease which is caused by bacteria selected from *Clostridium difficile*, *Clostridium perfringens* and *Staphylococcus aureus*.
xxii) According to one aspect of embodiment xxi), the present invention will relate to the use of a compound of formula I as defined in one of embodiments i) to vii), or of a pharmaceutically acceptable salt of such a compound, for the manufacture of a medicament intended to treat an intestinal disease which is caused by bacteria selected from *Clostridium difficile*, *Clostridium perfringens* and *Staphylococcus aureus*.
xxiii) According to one preferred aspect of embodiment xxii), the intestinal disease intended to be treated will be caused by *Clostridium difficile* (notably by a toxin producing strain of *Clostridium difficile*).
xxiv) According to another aspect of embodiment xxi), the present invention will relate to the use of a compound of formula I as defined in one of embodiments i) to vii), or of a pharmaceutically acceptable salt of such a compound, for the manufacture of a medicament intended to prevent an intestinal disease which is caused by bacteria selected from *Clostridium difficile*, *Clostridium perfringens* and *Staphylococcus aureus*.
xxv) According to one preferred aspect of embodiment xxiv), the intestinal disease intended to be prevented will be caused by *Clostridium difficile* (notably by a toxin producing strain of *Clostridium difficile*).
xxvi) Preferably, the patients for which the medicament manufactured according to one of embodiments xxi) to xxv) will be intended will be patients treated with other antibiotics or with antiviral therapies, patients with an immunocompromised system such as cytotoxic chemotherapy or organ transplant patients, elderly (65 years or older) patients, or patients of intensive care units or of long-term care facilities.
xxvii) Another aspect of this invention relates to a use in preventing or treating in an animal (e.g. in a dog, a cat, a pig, a cow or a horse) an intestinal disease caused by bacteria selected from *Clostridium difficile*, *Clostridium perfringens* and *Staphylococcus aureus*, said use comprising the administration to said animal of an effective amount of a compound of formula I as defined in one of embodiment i) to vii), or of a pharmaceutically acceptable salt of such a compound, for a duration sufficient to treat the intestinal disease.
xxviii) According to one aspect of embodiment xxvii), the present invention will relate to a use in treating in an animal (e.g. in a dog, a cat, a pig, a cow or a horse) an intestinal disease which is caused by bacteria selected from *Clostridium difficile*, *Clostridium perfringens* and *Staphylococcus aureus*, said use comprising the administration to said patient of an effective amount of a compound of formula I as defined in one of embodiments i) to vii), or of a pharmaceutically acceptable salt of such a compound, for a duration sufficient to treat the intestinal disease.
xxix) According to another aspect of embodiment xxvii), the present invention will relate to a use in preventing in an animal (e.g. in a dog, a cat, a pig, a cow or a horse) an intestinal disease which is caused by bacteria selected from *Clostridium difficile*, *Clostridium perfringens* and *Staphylococcus aureus*, said use comprising the administration to said patient of an effective amount of a compound of formula I as defined in one of embodiments i) to vii), or of a pharmaceutically acceptable salt of such a compound, for a duration sufficient to prevent the intestinal disease.

The intestinal diseases intended to be prevented or treated according to embodiments i) to xxix) of this invention comprise notably diarrhea, colitis and pseudomembranous colitis. Preferably, said intestinal diseases will be caused by *Clostridium difficile* (and especially by a toxin producing strain of *Clostridium difficile*).

The most suitable administration route for the compounds of formula I used according to embodiments i) to xxix) of the present invention will be the oral route. Administration may be daily (e. g., one to four times daily) or may be less frequent (e. g., once every other day or once or twice weekly).

Though the exact administration doses of a compound of formula I as defined in one of embodiments i) to vii), or of a pharmaceutically acceptable salt thereof, will have to be determined by the treating physician or veterinarian, it is expected that an amount between 0.5 and 50 mg of compound of formula I or pharmaceutically acceptable salt thereof per kg of patient body weight per day (for example an amount between 1 and 5 mg of compound of formula I or pharmaceutically acceptable salt thereof per kg of patient body weight per day) given once or twice daily for a duration of 3 to 15 days (for example for a duration of 7 to 14 days) will be appropriate.

The production of pharmaceutical compositions containing compounds of formula I as defined in one of embodiments i) to vii) or pharmaceutically acceptable salts thereof can be effected in a manner which will be familiar to any person skilled in the art (see for example Remington, The Science and Practice of Pharmacy, 21st Edition (2005), Part 5, "Pharmaceutical Manufacturing" [published by Lippincott Williams & Wilkins]) by bringing the described compound of formula I or its pharmaceutically acceptable salts, optionally in combination with other therapeutically valuable substances, into a galenical administration form together with suitable, non-toxic, inert, therapeutically compatible solid or liquid carrier materials and, if desired, usual pharmaceutical adjuvants.

The compounds of formula I can be manufactured in accordance with the present invention using the procedures described hereafter.

### PREPARATION OF COMPOUNDS OF FORMULA I

### Abbreviations:

The following abbreviations are used throughout the specification and the examples:
- AcOH: acetic acid
- AD-mix α: 1,4-*bis*(dihydroquinine)phthalazine, K₃Fe(CN)₆, K₂CO₃ and K₂OsO₄.2H₂O
- AD-mix β: 1,4-*bis*(dihydroquinidine)phthalazine, K₃Fe(CN)₆, K₂CO₃ and K₂OsO₄.2H₂O
- Alloc: allyloxycarbonyl
- aq.: aqueous
- Boc: *tert*-butoxycarbonyl
- *t*-BuOK: potassium *tert*-butylate
- Cbz: benzyloxycarbonyl
- CDAD: *Clostridium difficile associated* diarrhea
- CFU: colony forming units
- CLSI: Clinical Laboratory Standards Institute
- DBU: 1,8-diazabicyclo[5.4.0]undec-7-ene
- DCM: dichloromethane
- DIAD: diisopropyl azodicarboxylate
- DIPEA: *N*,*N*-diisopropylethylamine
- DMF: *N*,*N*-dimethylformamide
- DMSO: dimethylsulfoxide
- EA: ethyl acetate
- EDC: 1-(dimethylaminopropyl)-3-ethylcarbodiimide hydrochloride
- ESI: Electron Spray Ionisation
- ether or Et₂O: diethyl ether
- FC: flash chromatography
- h: hour
- Hex: *n*-hexane
- IC50: concentration that reduces the effect by 50%
- LZD-R: linezolid-resistant
- MeCN: acctonitrile
- MCPBA: *meta*-chloroperbenzoic acid
- MIC: minimum inhibitory concentration to inhibit bacterial growth
- MIC90: minimal inhibitory concentration to inhibit the growth of ≥90% of strains
- MRSA: methicillin resistant *Staphylococcus aureus*
- MeOH: methanol
- MS: Mass Spectroscopy
- NaOMe: sodium methylate
- NMP: *N*-methylpyrrolidinone
- OD595: optical density measured at 595 nM
- org.: organic
- Pd/C or Pd(OH)₂/C: palladium or dihydroxypalladium on charcoal
- PPh₃: triphenylphosphine
- rt: room temperature
- sat.: saturated
- SiO₂: silica gel
- TBDMSCI: *tert*-butyldimethylsilyl chloride
- TEA: triethylamine
- TFA: trifluoroacetic acid
- THF: tetrahydrofuran
- TMSCI: trimethylsilyl chloride

### General preparation methods:

The compounds of formula **I** can be manufactured in accordance with the present invention by
a) reacting the compound of formula II 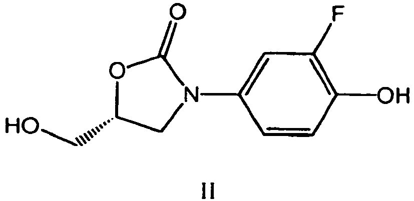 with a compound of formula III 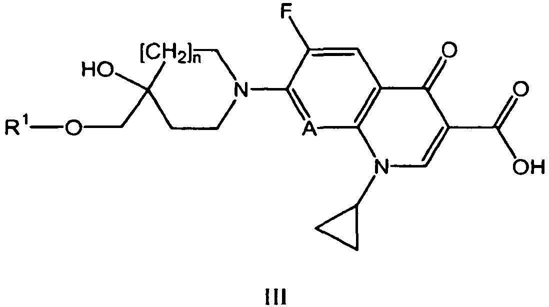 wherein n and A are as defined in formula I and R¹ is (C₁-C₃)alkylsulfonyl (e.g. methylsulfonyl), trifluoromethylsulfonyl or arylsulfonyl (e.g. phenyl- or p-tolylsulfonyl) preferably between about 10°C and 100°C (more preferably between about 40°C and 80°C), in the presence of an inorganic base such as K₂CO₃ or an organic base such as TEA in an organic solvent (e.g. DMF);
   or
b) reacting a compound of formula II with a compound of formula IV 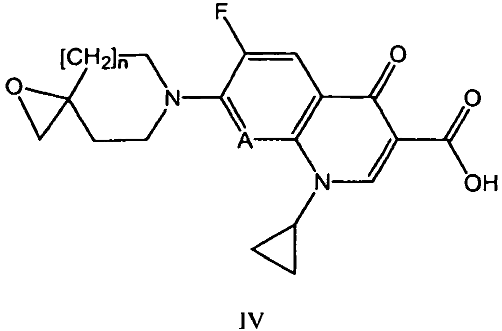 wherein n and A are as defined in formula I, as described in section a). Said compounds of formula IV can be obtained by treatment of compounds of formula III in the presence of an organic base (e.g. TEA) or an inorganic base (e.g. K₂CO₃ or an alkali methylate such as NaOMe or an alkali hydride such as NaH) in an organic solvent (e.g. DMF);
   or
c) reacting a compound of formula V 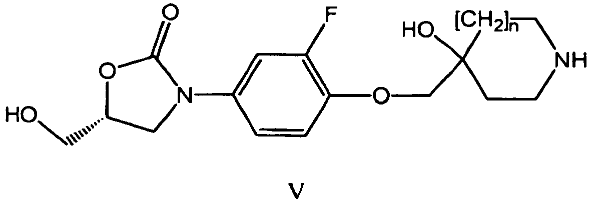 wherein n is as defined in formula I,
   with a compound of formula VI 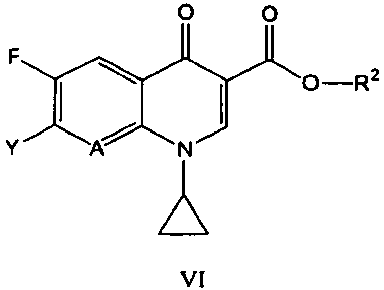 wherein A is as defined in formula I, Y is halogen and R² is hydrogen, BF₂ or B(OC(=O)(C₁-C₄)alkyl)₂, (C₁-C₅)alkyl (*e.g*. methyl, ethyl, *n*-propyl, *iso*-propyl or *tert-*butyl), allyl, aryl-(C₁-C₅)alkyl (*e.g*. benzyl, *p*-nitrobenzyl or *p*-methoxybenzyl), tri-(C₁-C₅)alkylsilyl (*e.g*. trimethylsilyl or *tert*-butyldimethylsilyl) or diaryl-(C₁-C₅)alkylsilyl (*e.g*. *tert*-butyldiphenylsilyl), preferably between about 10°C and 100°C, more preferably between about 40°C and 80°C in the presence of an organic base, such as TEA or DIPEA, in an organic solvent, e.g. NMP;
   or
d) converting a compound of formula VII 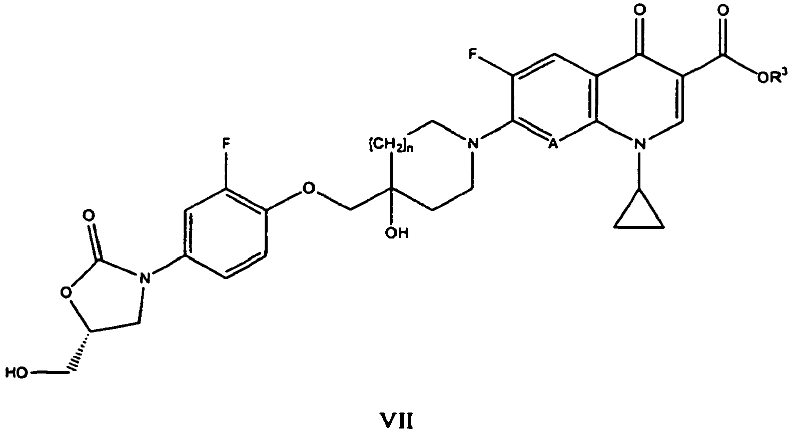 wherein R³ is (C₁-C₅)alkyl (*e.g*. methyl, ethyl, *n*-propyl, *iso*-propyl or *tert*-butyl), aryl-(C₁-C₅)alkyl (*e.g.* benzyl, *p*-nitrobenzyl or *p*-methoxybenzyl), allyl, tri-(C₁-C₅)alkylsilyl (e.g. trimethylsilyl or *tert*-butyldimethylsilyl) or diaryl-(C₁-C₅)alkylsilyl (e.g. *tert*-butyldiphenylsilyl) and n and A are as defined in formula I into the corresponding compound of formula I by hydrolysis, saponification or hydrogcnolysis (e.g. as reviewed in Protecting groups, Kocienski, P.J., Thieme (1994)).

Concerning the above process, the following should be noted: 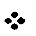
regarding variant a), the compound of formula III could also be replaced by an ester thereof, i.e. a compound of formula III_{E} 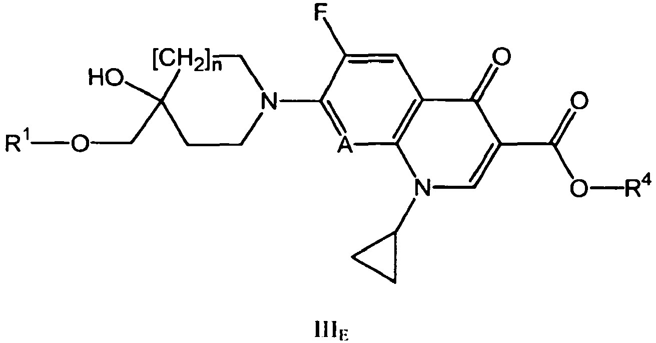 wherein n, A and R¹ are as defined in formula III and R⁴ represents alkyl, allyl or arylalkyl, in which case an ester deprotection step would follow the reaction of the compound of formula III_{E} with the compound of formula II (general methods to perform the ester deprotection step have been reviewed in Protecting groups, Kocienski, P.J., Thieme (1994)); 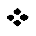
regarding variant a), the compound of formula II could also be replaced by a silyl ether thereof, i.e. a compound of formula II_{PG} 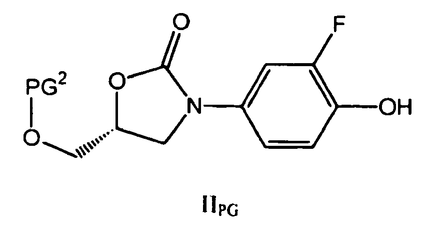 wherein PG² represents a silyl protecting group for an alcohol function such as a tri-(C₁-C₅)alkylsilyl (e.g. trimethylsilyl or *tert*-butyldimethylsilyl) or diaryl-(C₁-C₅)alkylsilyl (e.g. *tert*-butyldiphenylsilyl), in which case a deprotection step would follow the reaction of the compound of formula III or III_{E} with the compound of formula II_{PG} (general methods to perform such reactions have been reviewed in Protecting groups, Kocicnski, P.J., Thieme (1994)); 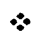
regarding variant c), the compound of formula V could also be replaced by a compound of formula V_{P} 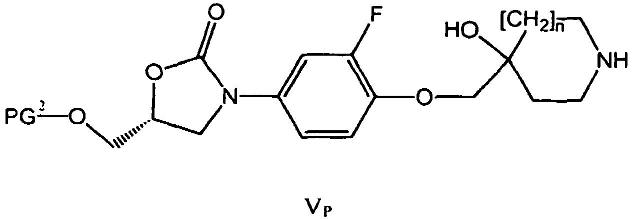 wherein n is as defined in formula V and PG² represents a protecting group for an alcohol function (e.g. an alkylsilyl or diarylalkylsilyl group such as trimethylsilyl, *tert-*butyldimethylsilyl or *tert*-butyldiphenylsilyl), in which case the appropriate deprotection step would follow the reaction of the compound of formula V_{P} with the compound of formula VI (general methods to perform such reactions have been reviewed in Protecting groups, Kocienski, P.J., Thieme (1994)); 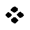
regarding variant c), when R² is not H, an additional ester deprotection step is required (general methods to perform such reactions have been reviewed in Protecting groups, Kocienski, P.J., Thieme (1994)), except for the cases wherein R² is BF₂ or B(OC(=O)(C₁-C₄)alkyl)₂ where the hydrolysis takes place already during the acidic work-up.

The compounds of formula I thus obtained may, if desired, be converted into their salts, and notably into their pharmaceutically acceptable salts.

Besides, whenever the compounds of formula I are obtained in the form of mixtures of enantiomers, the enantiomers can be separated using methods known to one skilled in the art (e.g. by formation and separation of diatereomeric salts or by chromatography over a chiral stationary phase). Whenever the compounds of formula I are obtained in the form of mixtures of diasteromers they may be separated by an appropriate combination of silica gel chromatography, HPLC and crystallization techniques.

### Preparation of the various synthesis intermediates:

### Preparation of the compound of formula II

The compound of formula II can be obtained by hydrogenation of the compound of formula VIII 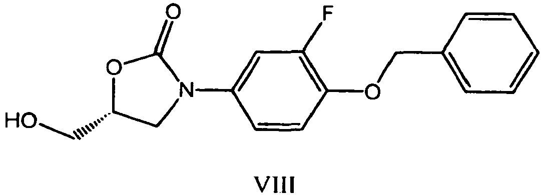 over a noble metal catalyst such as palladium or platinum on charcoal in a solvent such as THF, MeOH or EA between 0°C and 40°C or by hydrolysis of in presence of a solution of HBr in water or AcOH between 0°C and 80°C in a solvent such as AcOH.

### Preparation of the compounds of formula III

The compounds of formula III can be prepared as summarized in Scheme 1 hereafter.

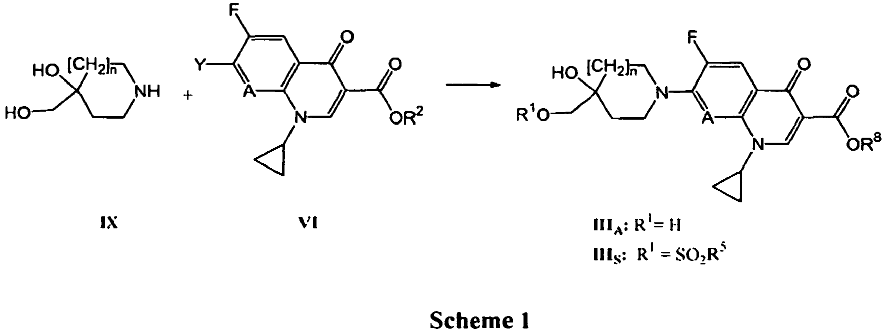

In Scheme 1, R² is H, alkyl, allyl or arylalkyl, and the other symbols are as before.

The compounds of formula III wherein R¹ is SO₂R⁵, R⁵ being alkyl, trifluoromethyl or aryl like phenyl or p-tolyl can be obtained (Scheme 1) from the compounds of formula III_{A} wherein R¹ is H by reaction with the corresponding sulfonyl chlorides in presence of an organic base such as TEA in a solvent such as DCM or THF between -10°C and 50°C. The compounds of formula III_{A} can be prepared by reaction of the compounds of formula VI with the piperidines or pyrrolidines of formula IX in the presence of an organic base such as TEA or DIPEA between 40°C and 100°C in a solvent such as THF, DMF or NMP. If R² is benzyl, the carboxylic acid of formula III_{S} can be liberated according to standard procedures as described in Protecting groups, Kocienski, P.J., Thieme (1994) (e.g. hydrogenation over Pd/C).

### Preparation of the compounds of formula V

The compounds of formula V can be prepared as summarized in Scheme 2 hereafter.

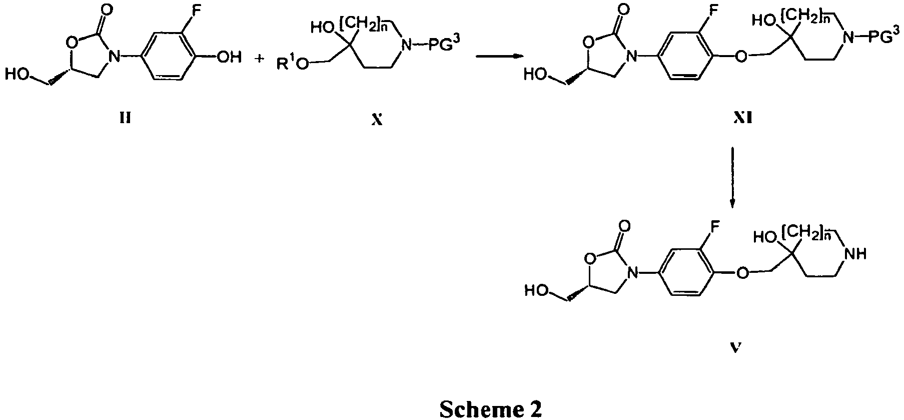

The compounds of formula V can be obtained by deprotecting the compounds of formula XI wherein PG³ represents a amino protecting group such as alkoxycarbonyl (e.g. Boc), benzyloxycarbonyl (e.g. Cbz), Alloc or benzyl. General methods to perform such protection/deprotection sequences of secondary amines have been reviewed in Protecting groups, Kocienski, P.J., Thieme (1994).

The compounds of formula XI can be obtained by reacting a compound of formula II with the compounds of formula X wherein R¹ is SO₂R⁵, R⁵ being alkyl, trifluoromethyl or aryl like phenyl or p-tolyl, or by reacting a compound of formula II with the corresponding epoxides derived from the compounds of formula X. Said epoxides can be obtained from the compounds of formula X, wherein R¹ is SO₂R⁵ upon treatment with either an organic base such as TEA, pyridine or DBU or an inorganic base such as K₂CO₃ in a solvent such as THF, ether or DCM between -10°C and 40°C. The compounds of formula X wherein R¹ is H are either commercially available (e.g. compounds X wherein PG³ = Boc, n = 1 and R¹ = H, or the epoxide derived from compounds of formula X wherein PG³ = Boc, n = 0) or can be made as explained later on. Alternatively, the compounds of formula XI can be obtained by reaction of the compound of formula II with the compounds of formula X wherein R¹ is H under Mitsunobu conditions.

### Preparation of the compounds of formula VI

The compounds of formula VI wherein R² is hydrogen, Me or Et are commercially available (e.g. compounds wherein A = CH, Y = Cl and R² = H, Me or Et, or Y = F and R²= BF₂, or compounds wherein A = N, Y = Cl and R² = H or Et). The compounds of formula VI wherein R² is B(OC(=O)(C₁-C₄)alkyl)₂ can be obtained from compounds of formula VI wherein R² is H according to WO 88/07998. The other compounds of formula VI can be obtained according to standard methods from compounds of formula VI wherein R² is H.

### Preparation of the compounds of formula VII

The compounds of formula VII can be obtained by coupling compounds of formula V or, alternatively, compounds of formula V_{P} as previously defined with compounds of formula VI as previously defined except that R² represents (C₁-C₅)alkyl (e.g. methyl, ethyl, *n*-propyl, *iso*-propyl or *tert*-butyl), aryl-(C₁-C₅)alkyl (e.g. benzyl, *p*-nitrobenzyl or p-methoxybenzyl), allyl, tri-(C₁-C₅)alkylsilyl (e.g. trimethylsilyl or *tert*-butyldimethylsilyl) or diaryl-(C₁-C₅)alkylsilyl (e.g. *tert*-butyldiphenylsilyl), under the same conditions as those described for the reaction of the compounds of formula V with the compounds of formula VI. If the compounds of formula V_{P} are used, the deprotection step can be carried out after the coupling reaction.

### Preparation of the compounds of formula VIII

The compound of formula VIII can be obtained according to WO 2004/096221.

### Preparation of the compounds of formula IX

The compounds of formula IX can be obtained by deprotection of compounds of formula X (R¹ = H), e.g. by treatment of the corresponding Boc protected compounds with TFA or by hydrogenation of the corresponding Cbz protected compounds over Pd/C.

### Preparation of the compounds of formula X

The compounds of formula X can be prepared from the methylidene derivatives of formula XII as summarized in Scheme 3 hereafter.

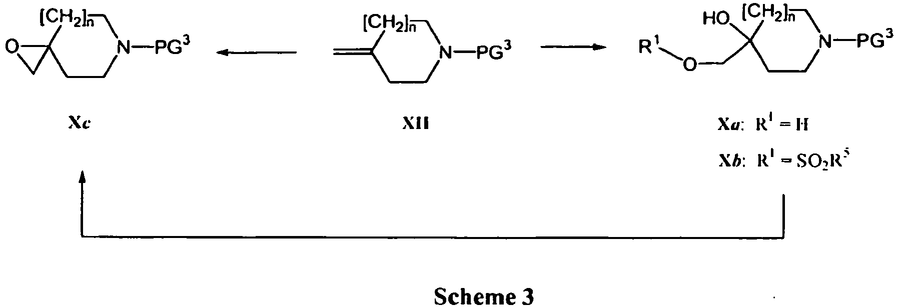

The compounds of formula X*b*, i.e. the compounds of formula X wherein R¹ is SO₂R⁵, are prepared from the corresponding compounds of formula X*a* wherein R¹ is H using the same methods used for the conversion of compounds of formula III_{A} into compounds of formula III_{S}. Compounds of formula Xa either are obtained from the known methylidene derivatives of formula XII (e.g. those wherein n = 0 and PG³ = benzyl, Boc or benzyloxycarbonyl - see EP 241206 and EP 550025; or those wherein n = I and PG³ = benzyl, Boc which are commercial) either by osmium tetroxide catalyzed *cis*-dihydroxylation or by its asymmetric version (Sharpless dihydroxylation using AD-mix α or β) as described in J. Am. Chem. Soc. (1988), 110, 1968. The compounds of formula X*c*, i.e. the epoxide derived from compounds of formula X*b* are obtained either by intramolecular ring closure of compounds of formula X*b* with an inorganic base such as K₂CO₃, or NaH or an organic base such as TEA or DBU, or by epoxidation of the methylidene double bond of compounds of formula XII with a peracid such as MCPBA. Alternatively, compounds of formula X*c* can also be obtained by reaction of the corresponding oxo derivatives (commercial when n = 0 or 1 and PG³ = Cbz or Boc) with trimethylsulfoxonium iodide or trimethylsulfonium iodide in presence of an alkali hydroxide such as potassium hydoxide in a polar solvent such as MeCN between 20 and 100°C (as described in J. Am. Chem. Soc. (1965), 87, 1353-1364 and Tetrahedron Lett. (1987), 28, 1877-1878).

Particular embodiments of the invention are described in the following Examples, which serve to illustrate the invention in more detail without limiting its scope in any way.

### EXAMPLES

All temperatures are stated in °C. All analytical and preparative HPLC investigations on non-chiral phases are performed using RP-C18 based columns. Analytical HPLC investigations are performed on two different instruments with cycle-times of ~2.5 min and ~3.5 min respectively. Unless otherwise stated, the values indicated for MS correspond to the main peaks ((M+H)⁺ with a variation of +/- 0.5 unit). In NMR spectra, coupling constants J are given in Hz.

### Standard work-up procedure:

After dilution in the appropriate org. solvent (see corresponding Example text), the org. phase is separated and sequentially washed with water and brine. In case of reaction performed in a water soluble solvent (e.g. MeOH, THF or DMF), the combined aq. layers are back-washed with the same solvent used to perform the workup. The combined org. phases are dried over MSO₄ and filtered. The filtrate is evaporated under reduced pressure.

### Standard chromatography procedure:

The crude material is dissolved in the minimum of eluent (see corresponding Example text) and chromatographed over SiO₂. The relevant fractions were pooled and evaporated under reduced pressure.

### Example 1: 1-cyclopropyl-6-fluoro-7-{4-[2-fluoro-4-((R)-5-hydroxymethyl-2-oxo-oxazolidin-3-yl)-phenoxymethyl]-4-hydroxy-piperidin-1-yl}-4-oxo-1,4-dihydro-quinoline-3-carboxylic acid:

### 1.i. (R)-3-(3-fluoro-4-hydroxy-phenyl)-5-hydroxymethyl-oxazolidin-2-one:

A solution of *(R)*-3-(4-benzyloxy-3-fluoro-phenyl)-5-hydroxymethyl-oxazolidin-2-one (6.34 g, prepared according to WO 2004/096221) in THF/MeOH (1:1; 200 ml) was hydrogenated over Pd/C 10% (1 g) overnight. The catalyst was filtered off, the filtrate evaporated under reduced pressure and the residue stirred in EA. The crystals were collected by filtration, affording 3.16 g (70% yield) of a colourless solid.
¹H NMR (DMSO_{d6}; δ ppm): 3.5 (m, 1H), 3.64 (m, 1H), 3.74 (dd, J = 8.8, 6.4, 1H), 3.99 (t, J=8.8, 1H), 4.64 (m, 1H), 5.16 (t, J=5.6, 1H), 6.93 (dd, J = 9.7, 8.8, 1H), 7.08 (ddd, J = 8.8, 2.6, 1.2, 1H), 7.45 (dd, J = 13.5, 2.6, 1H), 9.66 (s, I H).
MS (ESI): 228.1.

### 1.ii. 4-[2-fluoro-4-((R)-5-hydroxymethyl-2-oxo-oxazolidin-3-yl)-phenoxymethyl]-4-hydroxy-piperidine-1-carboxylic acid benzyl ester:

A solution of intermediate 1.i (1.27 g) and 1-oxa-6-aza-spiro[2.5]octane-6-carboxylic acid benzyl ester (1.60 g; prepared according to US 4244961) were dissolved in DMF (15 ml) and treated with Na₂CO₃ (1.16 g). The mixture was heated at 100°C overnight. The residue obtained after workup (DCM) was stirred in EA, and the solid was collected by filtration and sequentially washed with EA and Hex, affording 2.52 g (94.5% yield) of a beige solid.
   ¹H NMR (DMSO_{d6}; δ ppm): 1.57 (m, 4H), 3.14 (m, 2H), 3.54 (m, 1H), 3.64 (m, 1H), 3.79 (m, 5 H), 4.03 (t, J = 9.1,1 H), 4.66 (m, 1 H), 4.78 (s, I H), 5.05 (s, 2 H), 5.16 (t, J = 5.6, 1 H), 7.18 (m, 2 H), 7.32 (m, 5 H), 7.55 (d, J = 12, 1 H).
   MS (ESI): 475.0.

### 1.iii. (R)-3-[3-fluoro-4-(4-hydroxy-piperidin-4-ylmethoxy)-phenyl]-5-hydroxymethyl-oxazolidin-2-one:

A suspension of intermediate 1.ii (2.5 g) in EA/MeOH (1:1; 100 ml) was hydrogenated over Pd/C for 48 h. The suspension was heated at 40°C and the catalyst was filtered off.
The filtrate was evaporated under reduced pressure affording 1.61 g (89% yield) of a yellow powder.
¹H NMR (DMSO_{d6}; δ ppm): 1.4-1.63 (m, 4H), 2.67 (m, 2H), 2.83 (m, 2H), 3.53 (dd, J=4.0, 12.0, 1H); 3.66 (dd, J = 3.3, 12.0, 1H),3.71 (s, 2H); 3.80 (m, 1 H), 4.05 (t, J = 9.0, 1H), 4.48 (s, 1H), 4.68 (m, 1H), 5.20 (s, 1H), 7.20 (m, 2H), 7.57 (d, I H).
MS (ESI): 341.5.

### 1.iv. 1-cyclopropyl-6-fluoro-7-{4-[2-fluoro-4-((R)-5-hydroymethyl-2-oxo-oxazolidin-3-yl)-phenoxymethyl]-4-hydroxy-piperidin-1-yl}-4-oxo-1,4-dihydro-quinoline-3-carboxylic acid:

A solution of intermediate 1.iii (200 mg), 7-chloro-1-cyclopropyl-6-fluoro-1,4-dihydro-4-oxo-3-quinolinecarboxylic acid boron diacetate complex (241 mg; prepared according to WO 88/07998) and DIPEA (100 µl) in NMP (2 ml) was stirred at 85°C for 5 h. The reaction mixture was evaporated under reduced pressure and the residue was taken up in 5M HCl in MeOH (3 ml) and stirred. The resulting solid was collected by filtration and washed with MeOH to afford 230 mg (67% yield) of a yellow solid.
¹H NMR (DMSO_{d6}; δ ppm): 1.66-1.35 (m, 4H), 1.75 (d, J = 12.8, 2H), 1.95 (m, 2H), 3.33 (t broad, J = 11.0, 2H), 3.57 (m, 3H), 3.67 (dd, J = 12.3, 3.3, 1H), 3.83 (m, 2H), 3.92 (s, 2H), 4.06 (t, J=9.0, 1H), 4.69 (m, 1H), 7.24 (m, 2H), 7.60 (m, 2H), 7.90 (d, J = 13.3, 1H), 8.66 (s, 1H).
MS (ESI): 585.9.

### Example 2: 1-cyclopropyl-6-fluoro-7-{4-[2-fluoro-4-((R)-5-hydroxymethyl-2-oxo-oxazolidin-3-yl)phenoxymethyl]-4-hydroxy-piperidin-1-yl}-4-oxo-1,4-dihydro-[1,8]naphthyridine-3-carboxylic acid:

A solution of 7-chloro-1-cyclopropyl-6-fluoro-1,4-dihydro-4-oxo-1,8-naphthyridine-3-carboxylic acid (166 mg; commercial) and intermediate 1.iii (200 mg) in NMP (5 ml) was treated with TEA (0.32 ml) and TMSCI and heated at 85°C for 5 h. The reaction mixture was evaporated under reduced pressure and the residue was taken up in 5*M* HCL in MeOH (3 ml) and stirred for 30 min. The solution was evaporated under reduced pressure and the residue was taken up in EA. The resulting solid was collected by filtration and washed with EA, affording 271 mg (78% yield) of a yellow solid.
¹H NMR (DMSO_{d6}; δ ppm): 0.89-1.27 (m, 4H); 1.78 (d, J = 12.8, 2H); 1.90-2.04 (m, 2H); 3.53-3.88 (m, 6H); 3.88 (s, 2H), 4.06 (t, J = 9.0, 1H), 4.42 (d broad, J = 13.2, 2H), 4.44 (m, 1H); 7.11 (m, 2H); 7.55 (d, J = 14.5, 1H); 8.05 (d, J = 13.5, 1H); 8.60 (s, 1H).
MS (ESI): 586.8.

### Example 3: 1-cyclopropyl-6-fluoro-7-{(RS)-3-12-fluoro-4-((R)-5-hydroxymethyl-2-oxo-oxazolidin-3-yl)-phenoxymethyl]-3-hydroxy-pyrrolidin-1-yl}-4-oxo-1,4-dihydro-quinoline-3-carboxylic acid:

### 3.i. Diallyl-carbamic acid benzyl ester:

Benzoyl chloride (15.5 ml) was added dropwise over 30 min to a solution of diallylamine (12.3 ml) and TEA (21 ml) in DCM (100 ml) at 0°C. The reaction mixture was stirred at rt for 16 h. The residue obtained after work up (DCM) was purified by chromatography (Hex/EA 95:5) to give 20.71 g (88% yield) of a colourless liquid.
¹H NMR (DMSO_{d6}; δ ppm): 3.83 (dt, J = 1 and J = 6, 4H); 5.05-5.18 (m, 6H); 5.70-5.86 (m, 2H); 7.27-7.48 (m, 5H).

### 3.ii. 2,5-dihydro-pyrrole-1-carboxylic acid benzyl ester:

Benzylidene-bis(tricyclohexylphosphine)dichlororuthenium (5 g) was added to a solution of intermediate 3.i (17.56 g) in DCM (1.5 1) at rt under nitrogen. The reaction mixture was stirred at 40 °C for 2 h and concentrated *in vacuo.* The residue was purified by FC (Hex/EA 90:10) to give 14.08 g (91% yield) of a yellow liquid.
¹H NMR (DMSO_{d6}; δ ppm): 4.05-4.16 (m, 4H); 5.08 (s, 2H); 5.81-5.92 (m, 2H); 7.27-7.41 (m,5H).

### 3.iii. (RS)-3-hydroxy-pyrrolidine-1-carboxylic acid benzyl ester:

A 1M solution of borane in THF (9 ml) was added to a solution of intermediate 3.ii (1.81 g) in THF (25 ml) at 0 °C under nitrogen. The reaction mixture was stirred at rt for 16 h and was cooled to 0 °C. 20% NaOH (1.8 ml) was carefully added dropwise followed by 35% aq. hydrogen peroxide (1.2 ml). The mixture was stirred at 0 °C for 30 min and at rt for 2 h. Et₂O and an aq. 40% sodium bisulfite solution were added and the reaction mixture stirred vigorously for 15 min. The residue obtained after work up (Et₂O) was purified by FC (Hex/EA 5:5 to 3:7) to give 1.01 g (51 % yield) of a colourless oil.
¹H NMR (DMSO_{d6}; δ ppm): 1.67-1.82 (m, 1H); 1.82-1.96 (m, 1H); 3.16-3.25 (m, 1H); 3.28-3.44 (m, 3H); 4.20-4.29 (broad, 1H); 4.92 (d, J=3, 1H); 5.06 (s, 2H); 7.27-7.41 (m, 5H).

### 3.iv. 3-oxo-pyrrolidine-1-carboxylic acid benzyl ester:

A solution of intermediate 3.iii (1.10 g) in DCM (8 ml) was cooled to 0 °C and DIPEA (2.5 ml) was added dropwise, followed by a solution of sulfur trioxidc pyridine complex (1.79 g) in DMSO (6.5 ml). The reaction mixture was stirred at 0 °C for I h and was quenched by the addition of water (6 ml). The aq. layer was extracted with Et₂O/Hex (1:1, 3 x 5 ml) and the combined org. layers were concentrated *in vacuo.* The residue obtained after work up (Et₂O/Hex 1:1) was purified by FC (Hex/EA 5:5) to give 1.05 g (96% yield) of a yellowish oil.
¹H NMR (DMSO_{d6}; δ ppm): 2.48-2.61 (m, 2H); 3.61-3.80 (m, 4H); 5.09 (s, 2H); 7.27-7.41 (m, 5H).

### 3.v. 3-methylene-pyrrolidine-1-carboxylic acid benzyl ester:

t-BuOK (617 mg) was added in one portion to a white suspension of methyl triphenylphosphonium bromide (1.98 g) in THF (10 ml) at rt under nitrogen. The yellow suspension was stirred at rt for 1 h and then cooled to -10°C. A solution of intermediate 3.iv (1.05 g) in THF (2 ml) was added dropwise over 10 min and the reaction mixture was allowed to warm to rt over 2 h. The reaction was quenched by the addition of sat. aq. NH₄Cl (1ml) and diluted with EA. The residue obtained after work up (EA) was purified by chromatography (Hex/EA 90:10) to give 633 mg (64% yield) of a yellowish liquid.
¹H NMR (DMSO_{d6}; δ ppm): 2.48-2.61 (m, 2H); 3.36-3.53 (m, 2H); 3.84-4.01 (m, 2H); 4.97-5.03 (m, 2H); 5.08 (s, 2H); 7.27-7.41 (m, 5H).

### 3.vi. 1-oxa-5-aza-.spiro(2.4]heptane-5-carboxylic acid benzyl ester:

A solution of intermediate 3.v (7.21 g) in DCM (400 ml) was treated with MCPBA (20.1 g) and NaHCO₃ (22.3 g) at rt. The reaction was stirred at rt for 2 h, diluted with DCM (200 ml) and poured in a solution of Na₂SO₃ (45 g) in water (400 ml). The mixture was stirred for 10 min and the org. layer was separated. The residue obtained after work up (DCM) was purified by FC (Hex/EA 6:4) to give 4.37 g (56% yield) of a yellow oil.
¹H NMR (DMSO_{d6}; δ ppm): 1.70-1.83 (m, 1H); 2.22-2.37 (m, 1H); 2.90-2.94 (m, 1H); 2.95-2.99 (m, 1H); 3.15 (t, J = 11, 1H); 3.39-3.77 (m, 3H); 5.09 (s, 2H); 7.27-7.41 (m, 5H).

### 3.vii. (RS)-3-[2-fluoro-4-((R)-5-hydroxymethyl-2-oxo-oxazolidin-3-yl)-phenoxymethyl-3-hydroxy-pyrrolidine-1-carboxy/ic acid benzyl ester:

K₂CO₃ (274 mg) was added to a suspension of intermediate 1.i (300 mg) and intermediate 3.vi (338 mg) in DMF (3 ml). The reaction mixture was stirred at 80°C for 3 h and the solvent was removed *in vacuo.* The residue obtained after work up (DCM) was purified by FC (DCM/MeOH 95:5) to give 531 mg (87% yield) of a beige foam.
¹H NMR (DMSO_{d6}; δ ppm): 1.80-1.92 (m, 1H); 1.96-2.08 (m, 1H); 3.32-3.59 (m, 5H); 3.66 (ddd, J = 3, J = 6 and J = 13, 1H); 3.80 (dd, J = 6 and J = 9, 1H); 3.97-4.09 (m, 3H); 4.64-4.72 (m, 1H); 5.07 (s, 2H); 5.19 (t, J=6, 1H); 5.23 (s, 1H); 7.18-7.23 (m, 2H); 7.27-7.38 (m, 5H); 7.57 (dd, J = 2 and J = 14, 1H).
MS (ESI): 460.9.

### 3. viii. (R)-3-[3-fluoro-4-((RS)-3-hydroxy-pyrrolidin-3-ylmethoxy)-phenyl]-5-hydroxymethyl-oxazolidin-2-one:

A solution of intermediate 3.vii (259 mg) in THF/MeOH (1:1; 20 ml) was hydrogenated over 10% Pd/C (60 mg) for 20 h at rt. The reaction mixture was concentrated *in vacuo,* taken in DCM/MeOH 90:10 (20 ml) and stirred at rt for 30 min. The catalyst was filtered off and the filtrate concentrated *in vacuo* to give 184 mg (100% yield) of an orange foam.
MS (ESI): 327.3.

### 3.ix. 1-cyclopropyl-6-fluoro-7-{(RS)-3-[2-fluoro-4-((R)-5-hydroxymethyl-2-oxo-oxazo/idin-3-yl)-phenoxymethyl)-3-hydroxy-pyrrolidin-1-yl}-4-oxo-1.4 dihydro-quinoline-3-carboxylic acid:

A solution of intermediate 3.viii (226 mg) and 7-chloro-1-cyclopropyl-6-fluoro-1,4-dihydro-4-oxo-3-quinolinecarboxylic acid boron diacetate complex (270 mg; prepared according to WO 88/07998) in NMP (5 ml) was treated with DIPEA (120 µl) and stirred at 60°C for 2 h. The reaction mixture was concentrated *in vacuo* and the residue was taken in 5*M* HCl in MeOH (2 ml). The solution was stirred at rt for 1 h, concentrated *in vacuo* and the residue was purified by FC (DCM/MeOH/AcOH 95:4:1 to 90:9:1). The foamy residue was taken in MeOH (2 ml), stirred for 1 h and filtered. The crystals were collected and dried *in vacuo* to afford 23 mg (6% yield) of a beige solid.
¹H NMR (DMSO_{d6}; δ ppm): 1.10-1.34 (m, 4H); 1.98-2.10 (m, 1H); 2.14-2.26 (m, 1H); 3.48-3.70 (m, 3H); 3.71-3.89 (m, 5H); 4.05 (t, J = 9, 1H); 4.09-4.18 (m, 2H); 4.66-4.74 (m, 1H); 5.19 (t, J = 6, 1H); 5.40 (s, I H); 7.09 (d, J = 8, 1H); 7.18-7.3 1 (m, 2H); 7.59 (dd, J = 2 and J = 14, 1H); 7.82 (d, J = 14, 1H); 8.59 (s, I H); 15.52 (s, I H).
MS (ESI): 572.3.

### Example 4: antibacterial in vitro activity of the invention compounds against reference strains of Clostridium difficile or Clostridium perfringens:

### 4.i. Experimental method:

Minimal inhibitory concentrations (MICs) were determined by a broth microdilution assay. Brucella broth supplemented with a final concentration of 0.5 mg/L of Vitamin K₁ and 5 mg/L hemin was used as the test medium. Briefly, stock solutions of the compounds were prepared in DMSO (5.12 mg/ml), and 5 µl of a range of two-fold serial dilutions in 50% DMSO/50% H₂O were dispensed into 96-well microtiter plates containing 45 µl supplemented Brucella broth and shaken for 5 min. To prepare the inocula 24 h old colonies of *C*. *difficile* grown on Brucella agar supplemented with 5% laked sheep blood, 5 µg of hemin/ml, and 1 µg of vitamin K₁/ml were suspended in supplemented Brucella broth and adjusted to a density matching 0.5 McFarland standard. 50 µl of a 50-fold dilution of this suspension was used to inoculate the wells of the 96-well plate resulting in approximately 10⁴ colony forming units (CFU) per well. The final DMSO concentration was 2.5%. The final concentration range was 0.03 - 16 µg /mL. The plates were incubated under anaerobic conditions for 48h at 37°C. After incubation the plates were read in a plate reader at OD₅₉₅ (Ultramark, Biorad Laboratories). The MICs were initially read at the lowest concentration showing >90% of growth inhibition compared to control wells. Plates were also checked visually with the aid of a reading mirror, and MICs were confirmed by the absence of visual growth.

### 4.ii. Results:

The compounds of Examples 1-3 were tested *in vitro* for their activity to inhibit growth of three reference strains of *C*. *difficile* or *Clostridium perfringens.* The results obtained are summarized in Table 1 hereafter.

**Table 1**

| Compound tested | MIC regarding C. *difficile* ATCC 43596 (µg/ml) | MIC regarding C. *difficile* ATCC 9689 (µg/ml) | MIC regarding C. *difficile* NC 13366 (µg/ml) | MIC regarding C. *perfringens* DSM 756 (µg/ml) |
|---|---|---|---|---|
| Ciprofloxacin | 8 | 8 | > 16 | 0.25 |
| Linezolid | 8 | 2 | 1 | 4 |
| Example 1 | 0.125 | 0.125 | 0.06 | ≤ 0.03 |
| Example 2 | 0.06 | 0.125 | 0.03 | ≤ 0.03 |
| Example 3 | 0.25 | 0.5 | 0.25 | 0.125 |

The compounds of Examples 1-3 showed potent *in vitro* activity against the strains of *C*. *difficile* and *C*. *perfringens* tested. Strong activity was also observed against the quinolone-rcsistant hypervirulent strain NC13366. The compounds of Examples 1 and 2 were slightly more active than the compound of Example 3 and all Example compounds were clearly more active than ciprofloxacin and linezolid.

### Example 5: antibacterial in vitro activity of the compound of Example 1 against a collection of clinical isolates of Clostridium difficile:

### 5.i. Experimental method:

The CLSI-recommended reference agar dilution method for anaerobes (M 11-A6) was used for susceptibility testing. Brucella agar supplemented with 5% laked sheep blood, 5 µg of hemin/ml, and 1 µg of vitamin K₁/ml was the test medium. The test compounds were serially diluted and added to the molten supplemented agar. Growth controls were done on drug free plates. Prior to testing, all isolates were subcultured twice onto enriched brucella agar plates. Bacterial colonies were suspended in brucella broth. Standardization with a Vitek colorimeter was used to prepare each inoculum to the equivalent of a 0.5 McFarland standard, approximating 10⁴-10⁵ CFU per spot after application with a Steers replicator. The plates were incubated under anaerobic conditions for 48 h at 37°C. The minimum inhibitory concentration (MIC) was the concentration that completely inhibited visible growth as compared to the drug-free control. All antibiotics were prepared and tested along with vancomycin and metronidazole as controls.

### 5.ii. Results:

The compound of Example I was tested for its activity to inhibit growth of a collection of 209 diverse clinical isolates of *C. difficile in vitro.* The compound of Example I showed a MIC90 (minimal inhibitory concentration to inhibit the growth of 90% of strains or more) of 0.25 µg/ml, the MICs obtained ranging from 0.06 to 0.5 µg/ml. It was on average more active than vancomycin, metronidazole and linezolid, which had MIC90s of 2, 1 and 8 µg/ml, respectively.

### Example 6: antibacterial in vitro activity of the invention compounds againstaerobic Gram-positive bacteria:

### 6.i. Experimental method:

Minimal inhibitory concentrations (MICs) were determined by a broth microdilution assay following the guidelines of the Clinical Laboratory Standards Institute [CLSI, formerly NCCLS, 1997]. Briefly, stock solutions of the compounds were prepared in DMSO (5.12 mg/ml), serially diluted in cation-adjusted Mueller-Hinton Broth 11 (CaMHB), and dispensed in microtiter plates with the help of a Biomek 2000 pipeting robot (Beckman Coulter). The final DMSO concentration was 2.5% or below. Plates were inoculated to achieve a concentration of generally 3-6x10⁵ CFU/ml. After incubation at 37°C for 18-24 h, the plates were read in a plate reader at OD₅₉₅ (Ultramark, Biorad Laboratories). The MICs were initially read at the lowest concentration showing >90% of growth inhibition compared to control wells. Plates were also checked visually with the aid of a reading mirror, and the MICs were confirmed by the absence of visual growth.

### 6.ii. Results:

The compound of Example 1 was tested against Gram-positive aerobic bacteria. The results obtained are summarised in Table 2 hereafter.

**Table 2**

| Strain | MIC measured for vancomycin (µg/ml) | MIC measured for ciprofloxacin (µg/ml) | MIC measured for linezolid (µg/ml) | MIC measured for the compound of Example 1 (µg/ml) |
|---|---|---|---|---|
| *S. aureus* ATCC 29213 | 1 | 0.25 | 2 | 0.25 |
| *S. aureus* A-798 (MRSA) | 1 | >16 | 2 | 0.25 |
| *S. aureus* ATCC 43300 (MRSA) | 1 | 0.25 | 2 | 0.25 |
| *S. aureus* S1 (LZD-R) | 1 | 0.25 | > 32 | 1 |
| *E. faecalis* ATCC 29212 | 1 | 2 | 2 | 0.25 |
| *E. faecalis* H 4060 | > 32 | > 16 | 2 | 0.25 |
| *E. faecalis* H 6279 | > 32 | > 16 | 2 | 0.25 |
| *E. faecalis* H 6897 | > 32 | > 16 | 4 | 1 |
| *E. faecalis* H 7094 | > 32 | > 16 | 4 | 0.5 |
| *E. faecalis* H 7460 | > 32 | > 16 | 16 | 1 |
| *E. faecium* ATCC 19434 | 1 | 8 | 2 | 0.25 |
| *E. faecium* H 9070 | > 32 | > 16 | 2 | 1 |
| *E. faecium* H 7969 | > 32 | > 16 | 4 | 1 |
| *E. faecium* H 7966 | > 32 | > 16 | 1 | 0.5 |
| *E. faecium* H 7965 | > 32 | > 16 | 2 | 0.5 |
| *E. faecium* H 7937 | > 32 | > 16 | 1 | 0.25 |
| *E. faecium* A 962 | > 32 | > 16 | 4 | 0.5 |
| *E. faecium* A 949 | > 32 | > 16 | 2 | 1 |
| *E. faecium* L1 (LZD-R) | 0.5 | > 16 | > 32 | 2 |

In summary, the compound of Example I had potent *in vitro* activity against strains of *Staphylococcus aureus, S. aureus* MRSA, *Enterococcus faecalis,* and *Enterococcus faecium.* It was active against strains resistant to vancomycin, ciprofloxacin or linezolid.

### Example 7: antibacterial in vitro activity of the compound of Example 1 against particular anaerobic bacterial strains:

Using the experimental methods described at Example 5 (see 5.i), the compound of Example 1 (Ex. 1) was tested *in vitro* for the ability to inhibit the growth of certain anaerobic bacteria that are known or suspected to play a role in the normal gut flora and in the maintenance of its role in protection against *C. difficile* overgrowth (hereafter collectively called "the commensal gut bacteria"). As references, the compound of Example 5 of WO 2005/058888 (R1) and ciprofloxacin (CP) were tested at the same time. The MICs thus obtained are shown in Table 3 hereafter.

**Table 3**

| Strains | | **MIC [µg/ml]** | | |
|---|---|---|---|---|
| | | **R1** | **Ex. 1** | **CP** |
| *Clostridium difficile* A- 1179 | NC 13366 | ≤0.06 | ≤0.06 | >32 |
| *Fusobacterium necrophorum* A-1260 | DSM 20698 | 1 | 1 | 4 |
| *Bacteroides ovatus* A-991 | ATCC 8483 | 8 | 16 | 16 |
| *Bacteroides fragilis* A-992 | ATCC 25285 | 2 | 8 | 8 |
| *Bacteroides thetaiotaomicron* A-990 | ATCC 29741 | 4 | 8 | 16 |
| *Bacteroides vulgatus* A-989 | ATCC 8482 | 1 | 2 | 32 |
| *Bacteroides fragilis* A-502 | T 7403 | 2 | 4 | 8 |
| *Bacteroides fragilis* A-348 | T 8673 | 1 | 2 | 8 |
| *Bacteroides fragilis* A-217 | B 6306 | 2 | 4 | 8 |
| *Bacteroides fragilis* A-501 | B 8039 | 4 | 4 | 32 |
| *Bacteroides fragilis* A-294 | T 9174 | 2 | 4 | 8 |
| *Bacteroides fragilis* A-293 | B 2518 | 2 | 4 | 8 |
| *Bacteroides fragilis* A-260 | T 9865 | 1 | 4 | 8 |
| *Eubacterium limosum* A-1259 | DSM 20698 | 0.125 | 1 | 2 |
| *Finegoldia magna* A-1254 | DSM 20470 | ≤0.06 | 0.25 | 2 |
| *Bifidobacterium adolescentis* A-1257 | DSM 20083 | ≤0.06 | 0.5 | 2 |
| *Bifidobacterium bifidum* A-1258 | DSM 20456 | 0.125 | 0.5 | 4 |
| *Lactobacillus acidophilus* A-1255 | DSM 20079 | ≤0.06 | 0.5 | 32 |
| *Lactococcus lactis* A-1256 | DSM 20069 | ≤0.06 | 0.5 | 1 |

In summary, the compound of Example 1 showed reduced activity regarding the commensal gut bacteria compared to *C. difficile.* For *Bacteroides* spp., which are important members of the human protective gut flora, the activity is 30 to 200 times lower. This selective activity offers the potential for selective killing of *C. difficile* in the gut while sparing important bacteria of the gut flora.

Besides, when compared to the compound of Example 5 of WO 2005/058888, the compound of Example 1 showed in most cases 2 to 4 times less activity against the tested *Bacteroides spp.,* 4 times less activity against the *Eubacterium limosum* A-1259 or *Finegoldia magna* A-1254 strains, 4 to 8 times less activity against the tested *Bifidobacterium* spp. and 8 times less activity against the *Lactobacillus acidophilus* A-1255 and *Lactococcus lactis* A-1256 strains.

Therefore, when compared to the compound of Example 5 of WO 2005/058888 (R1), the compound of Example 1 was significantly less active against the commensal gut bacteria, indicating an advantage for selectively killing of *C. difficile* in the gut.

### Example 8: effect of the compound of Example 1 on C. difficile toxin production:

### 8.i. Experimental method:

Hypertoxigenic *C. difficile* strain NC 13366 was grown anaerobically in supplemented Brucella broth for 24 h at 37°C to a cell density of approximately 1 x 10⁸ CFU/ml. Bacteria were washed by centrifugation and resuspended in the same volume of supplemented Brucella broth containing antibiotics at 1 and 8 µg/ml, respectively. The control contained no antibiotic. The cultures were further incubated anaerobically at 37°C. At day 5 culture supernatants were collected and assayed for toxin A and toxin B as described hereafter.

Toxin A was detected by Western Blotting using the NuPage Large Protein Analysis System (Invitrogen LP0001, according to kit instructions). The Western Breeze anti Mouse Immunodetection kit (Invitrogen WB7103) with anti CdTA mouse monoclonal antibodies (PCG4.1, Biodesign C70517M) was used.

*C. difficile* cytotoxic toxin (toxin B) was semiquantitatively detected by testing supernatants of *C. difficile* cultures for cell-rounding activity in CHO cells. CHO cells were seeded into 96-well flat bottom plates (1x10⁵ cells/well) and allowed to attach for 3 h.

Appropriate dilutions of filter sterilized supernatants were added to the cells and further incubated at 37°C, in 5% CO₂. After 20 h of incubation cell rounding was determined using an inverted microscope. The toxin titer was defined as the highest serial 10-fold dilution of the supernatant resulting in >90% cell rounding.

Colony forming units (CFU) were determined by plating appropriate dilutions of test samples on supplemented Brucella agar and colony count after 48 h incubation at 37°C under anaerobic conditions.

### 8.ii. Results:

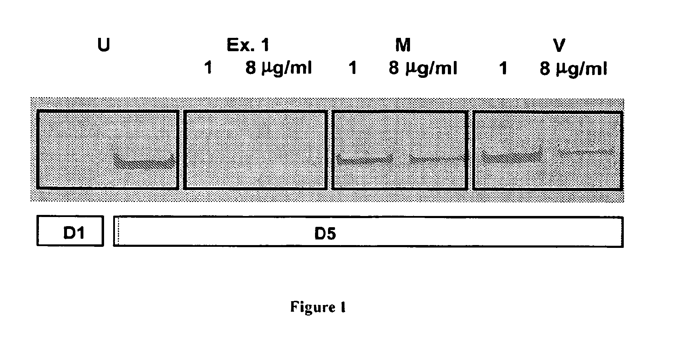

The outlook of a Western Blot plate to analyse toxin A production in supernatants of static high density cell cultures of *C. difficile* NC 13366 (a toxin-hyperproducing NAPI/027-type strain that caused outbreaks in hospitals with high mortality) is shown in Figure 1.

The experimental method mentioned at paragraph 8.i was used to obtain the result shown in Figure 1. At day 1 (D1), cultures were treated with either the compound of Example 1 (Ex. 1), vancomycin (V), metronidazole (M) at 1 and 8 µg/ml or left untreated (U); at day 5 (D5), the contents of toxin A in the supernatants were analysed and compared with those observed at D1 (see Figure 1). In high density five day old stationary phase cultures of toxigenic *C. difficile,* the compound of Example I inhibited *de novo* toxin A production at concentrations of 1 and 8 µg/ml, *i.e* at 2 and 16x the MIC (see Figure 1). In contrast, metronidazole and vancomycin did not inhibit toxin A production under these conditions or did so only weakly.

Culture supernatants were also analyzed for the cytotoxic toxin B by using the cell-based cell-rounding assay mentioned at paragraph 8.i. Activities of the supernatants of cultures treated with the compound of Example I were reduced to >90% compared to the untreated control. In contrast, activities of supernatants of vancomycin-treated cultures were not reduced (at I µg/ml) or reduced by only 30% (at 8 µg/ml).

In summary, the compound of Example 1 has the ability to stop efficiently the synthesis of *C. difficile* toxins A and B even in high density static cultures and in absence of killing, which is not the case for the vancomycin and metronidazole, the favoured drugs currently used for treatment of *C. difficile* infections.

### Example 9: effect of the compound of Example 1 on C. difficile spore formation:

### 9.i. Experimental method:

*C. difficile* A-1050 (ATCC 43596) was grown to logarithmic growth in supplemented Brucella broth resulting in turbidity matching McFarland 0.5 standard (6-7 h at 37°C under anaerobic conditions). Cultures were diluted 1:50 in tubes containing 1 ml of supplemented Brucella broth containing the antibiotics at various concentrations in 2-fold dilution steps. Tubes were further incubated for 4 days at 37°C under anaerobic conditions. The MIC was defined as the concentration that inhibited visible growth (turbidity) completely. 0.5x MIC was the highest concentration of drug allowing visible growth.

Assessment of colony forming units (CFUs) of total cells was performed by plating appropriate 10-fold dilutions of the cultures on Brazier's agar containing 4% egg yolk suspension and 1% lysed horse blood (Lab M). Colonies were counted after incubation at 37°C for 48 h under anaerobic conditions. Spore counts were done by selectively killing of vegetative cells by treatment with 50% ethanol for 1 h at rt and sub-sequent CFU count on Braziers' agar as detailed above.

### 9.ii. Results:

*C. difficile* is a spore forming organism and spores can persist for a long time in the hospital environment. Spores are very resistant to common disinfection procedures. Therefore spores play an important role in the transmission and persistence of *C. difficile* infections. Growing *in vitro* cultures of *C. difficile* were treated with various sub-MIC concentrations of the compound of Example I, vancomycin or metronidazole and investigated for the effect on the production of spores after 4 days of anaerobic incubation at 37°C (see paragraph 9.i). At a concentration representing 0.5x the MIC, treatment with compound of Example 1 resulted in low spore counts (<1% spores of total cell count). In contrast, untreated cultures and cultures treated with metronidazole or vancomycin at concentrations of 0.5x the MIC showed high sporulation rates (>90% spores of total cell counts). This indicates that the compound of Example I has the potential to reduce the production of spores during treatment of *C. difficile* infection more efficiently than vancomycin or metronidazole.

### Example 10: in vivo activity of an invention compound in the C. difficile hamster model:

### 10.i. Experimental method:

Golden Syrian hamsters were given a single injection of clindamycin phosphate (10 mg/kg s.c.), and a day later animals were infected by gavage with 10⁵ CFU of toxigenic *C difficile* strain 10465. A fulminant colitis results in the majority (∼95%) of animals and develops 1 to 2 days after the administration of *C. difficile.* Untreated, the colitis progresses rapidly and turns into severe colitis, hemorrhagic necrosis of the cecum and death. The compound of Example 1 was administered orally as a suspension at three dosage levels (10 mg/kg, 30 mg/kg and 100 mg/kg; n = 10 per group). Vancomycin (50 mg/kg) was used as a control. Animals were treated once daily for 5 days and afterwards maintain under observation for an additional 21 days.

The animals were observed three times daily for morbidity and presence or absence of diarrhoea. The final endpoint of the study was survival. Animals judged to be in a moribund state [extended period of weight loss progressing to an emaciated state, anorexia for 24-48 h, prolonged lethargy (more than 3 days), signs of paralysis, skin erosions or trauma, hunched posture, distended abdomen] were euthanized by a single injection of sodium pentobarbital.

### 10.ii. Results:

The therapeutic utility of the compound of Example 1 was tested using the protocol of paragraph 10.i. All the infected untreated animals died between 2 and 5 days after infection whereas all animals treated with 10, 30 or 100 mg/kg of compound of Example 1 survived during the 5 day treatment period. At the lowest dose level 40% of the animals survived the relapse phase of 21 days after treatment, whereas in the 30 and 100 mg/kg groups 80 and 100% of animals survived without relapse. Vancomycin with a 50 mg dose was used as a control. These results demonstrate that the compound of Example I has the capacity to treat animals with *C. difficile* infections comparable to vancomycin.

## Claims

1. A compound of formula I 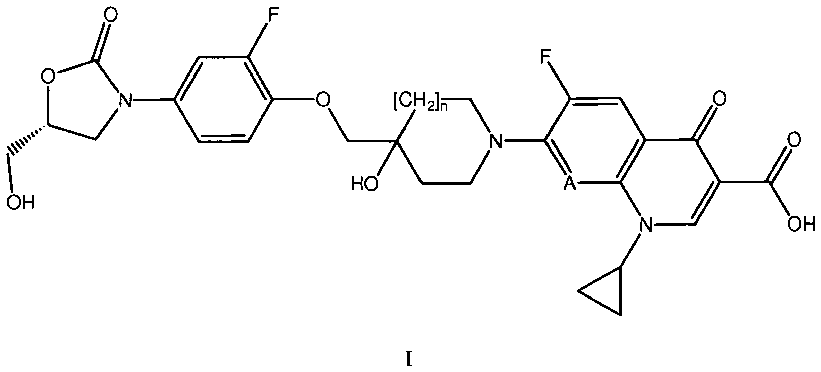 wherein
A is N or CH; and
n is 0 or 1;
or a pharmaceutically acceptable salt thereof, for use in preventing or treating intestinal diseases which are caused by bacteria selected from *Clostridium difficile, Clostridium perfringens* or *Staphylococcus aureus.*

2. A compound of formula I as defined in claim 1, or a pharmaceutically acceptable salt thereof, for the use mentioned in claim 1 wherein A is CH.

3. A compound of formula I as defined in claim 1 or 2, or a pharmaceutically acceptable salt thereof, for the use mentioned in claim 1 wherein n is 1.

4. A compound of formula I as defined in claim 1, which is selected from:
- 1-cyclopropyl-6-fluoro-7-{4-[2-fluoro-4-((R)-5-hydroxymethyl-2-oxo-oxazolidin-3-yl)-phenoxymethyl]-4-hydroxy-piperidin-1-yl}-4-oxo-1,4-dihydro-quinoline-3-carboxylic acid;
- 1-cyclopropyl-6-fluoro-7-{4-[2-fluoro-4-((*R*)-5-hydroxymethyl-2-oxo-oxazolidin-3-yl)-phenoxymethyl]-4-hydroxy-piperidin-1-yl}-4-oxo-1,4-dihydro-[1,8]naphthyridine-3-carboxylic acid; and
- 1-cyclopropyl-6-fluoro-7-{3-[2-fluoro-4-((*R)*-5-hydroxymethyl-2-oxo-oxazolidin-3-yl)-phenoxymethyl]-3-hydroxy-pyrrolidin-1-yl}-4-oxo-1,4-dihydro-quinoline-3-carboxylic acid;
or a pharmaceutically acceptable salt of such a compound, for use in preventing or treating intestinal diseases which are caused by bacteria selected from *Clostridium difficile, Clostridium perfringens* or *Staphylococcus aureus.*

5. A compound of formula I as defined in claim 1 or a pharmaceutically acceptable salt thereof, which is 1-cyclopropyl-6-fluoro-7-{4-[2-fluoro-4-((*R*)-5-hydroxymethyl-2-oxo-oxazolidin-3-yl)-phenoxymethyl]-4-hydroxy-piperidin-1-yl } -4-oxo-1,4-dihydro-quinoline-3-carboxylic acid or a pharmaceutically acceptable salt thereof, for use in preventing or treating intestinal diseases which are caused by bacteria selected from *Clostridium difficile, Clostridium perfringens* or *Staphylococcus aureus.*

6. A compound of formula I as defined in one of claims 1 to 5, or a pharmaceutically acceptable salt thereof, for use in preventing or treating the intestinal diseases which are caused by *Clostridium difficile.*

7. A compound of formula I as defined in one of claims 1 to 5, or a pharmaceutically acceptable salt thereof, for use in preventing or treating the intestinal diseases which are caused by a toxin producing strain of *Clostridium difficile.*

8. A compound of formula I as defined in one of claims 1 to 5, or a pharmaceutically acceptable salt thereof, for use in preventing or treating diarrhea diseases associated with enterotoxigenic strains of *Clostridium difficile, Clostridium perfringens* or *Staphylococcus aureus* without increasing the concentration of vancomycin-resistant enterococci in the gut.

9. A compound of formula I as defined in one of claims 1 to 5, or a pharmaceutically acceptable salt thereof, for use in preventing or treating diarrhea diseases associated with enterotoxigenic strains of *Clostridium difficile, Clostridium perfringens* or *Staphylococcus aureus* and reducing the concentration of vancomycin-resistant enterococci in the gut.

10. Use of a compound of formula I as defined in one of claims 1 to 5, or of a pharmaceutically acceptable salt of such a compound, for the manufacture of a medicament intended to prevent or treat an intestinal disease which is caused by bacteria selected from *Clostridium difficile, Clostridium perfringens* and *Staphylococcus aureus.*

11. Use according to claim 10, wherein the medicament manufactured is intended to treat the intestinal disease.

12. Use according to claim 10, wherein the medicament manufactured is intended to prevent the intestinal disease.

13. Use according to claim 10, wherein the medicament manufactured is also intended not to increase the concentration of vancomycin-resistant enterococci in the gut.

14. Use according to claim 10, wherein the medicament manufactured is also intended to reduce the concentration of vancomycin-resistant enterococci in the gut.

## Patentansprüche

1. Verbindung der Formel I 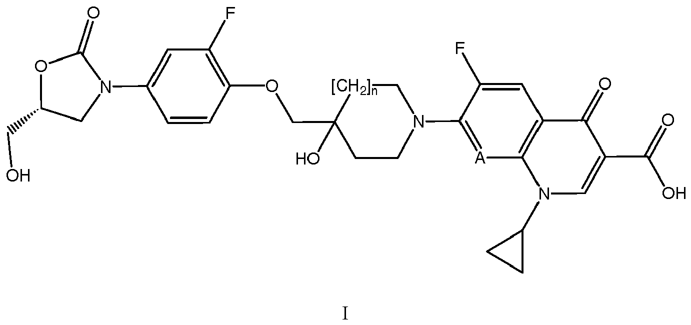 wobei
A N oder CH ist; und
n 0 oder 1 ist;
oder ein pharmazeutisch akzeptables Salz davon, für eine Verwendung zur Verhütung oder Behandlung von Darmerkrankungen, die durch Bakterien verursacht werden, die ausgewählt sind aus *Clostridium difficile, Clostridium perfringens* oder *Staphylococcus aureus.*

2. Verbindung der Formel I nach Anspruch 1, oder ein pharmazeutisch akzeptables Salz davon, für die in Anspruch 1 erwähnte Verwendung, wobei A CH ist.

3. Verbindung der Formel I nach Anspruch 1 oder 2, oder ein pharmazeutisch akzeptables Salz davon, für die in Anspruch 1 erwähnte Verwendung, wobei n 1 ist.

4. Verbindung der Formel I nach Anspruch 1, die ausgewählt ist aus:
- 1-Cyclopropyl-6-fluor-7-{4-[2-fluor-4-((*R*)-5-hydroxymethyl-2-oxo-oxazolidin-3-yl)-phenoxymethyl]-4-hydroxy-piperidin-1-yl } -4-oxo-1,4-dihydro-chinolin-3-carbonsäure;
- 1-Cyclopropyl-6-fluor-7-{4-[2-fluor-4-((*R*)-5-hydroxymethyl-2-oxo-oxazolidin-3-yl)-phenoxymethyl]-4-hydroxy-piperidin-1-yl} -4-oxo-1,4-dihydro-[1,8]naphthyridin-3-carbonsäure; und
- 1-Cyclopropyl-6-fluor-7-{3-[2-fluor-4-((*R*)-5-hydroxymethyl-2-oxo-oxazolidin-3-yl)-phenoxymethyl]-3-hydroxy-pyrrolidin-1-yl} -4-oxo-1,4-dihydro-chinolin-3-carbonsäure;
oder ein pharmazeutisch akzeptables Salz einer solchen Verbindung, für eine Verwendung zur Verhütung oder Behandlung von Darmerkrankungen, die durch Bakterien verursacht werden, die ausgewählt sind aus *Clostridium difficile, Clostridium perfringens* oder *Staphylococcus aureus.*

5. Verbindung der Formel I nach Anspruch 1, oder ein pharmazeutisch akzeptables Salz davon, die 1-Cyclopropyl-6-fluor-7-{4-[2-fluor-4-((*R*)-5-hydroxymethyl-2-oxo-oxazolidin-3-yl)-phenoxymethyl]-4-hydroxy-piperidin-1-yl}-4-oxo-1,4-dihydro-chinolin-3-carbonsäure oder ein pharmazeutisch akzeptables Salz davon ist, für eine Verwendung zur Verhütung oder Behandlung von Darmerkrankungen, die durch Bakterien verursacht werden, die ausgewählt sind aus *Clostridium difficile, Clostridium perfringens* oder *Staphylococcus aureus.*

6. Verbindung der Formel I nach einem der Ansprüche 1 bis 5, oder ein pharmazeutisch akzeptables Salz davon, für eine Verwendung zur Verhütung oder Behandlung von Darmerkrankungen, die durch *Clostridium difficile* verursacht werden.

7. Verbindung der Formel I nach einem der Ansprüche 1 bis 5, oder ein pharmazeutisch akzeptables Salz davon, für eine Verwendung zur Verhütung oder Behandlung von Darmerkrankungen, die durch einen toxinproduzierenden Stamm von *Clostridium difficile* verursacht werden.

8. Verbindung der Formel I nach einem der Ansprüche 1 bis 5, oder ein pharmazeutisch akzeptables Salz davon, für eine Verwendung zur Verhütung oder Behandlung von Durchfallerkrankungen, die mit enterotoxinogenen Stämmen von *Clostridium difficile, Clostridium perfringens* oder *Staphylococcus aureus* assoziiert sind, ohne dass die Konzentration von vancomycinresistenten Enterokokken im Darm erhöht wird.

9. Verbindung der Formel I nach einem der Ansprüche 1 bis 5, oder ein pharmazeutisch akzeptables Salz davon, für eine Verwendung zur Verhütung oder Behandlung von Durchfallerkrankungen, die mit enterotoxinogenen Stämmen von *Clostridium difficile, Clostridium perfringens* oder *Staphylococcus aureus* assoziiert sind, wobei die Konzentration von vancomycinresistenten Enterokokken im Darm verringert wird.

10. Verwendung einer Verbindung der Formel I nach einem der Ansprüche 1 bis 5, oder eines pharmazeutisch akzeptablen Salzes einer solchen Verbindung, zur Herstellung eines Medikaments, das für die Verhütung oder Behandlung einer Darmerkrankung vorgesehen ist, die durch Bakterien verursacht wird, die ausgewählt sind aus *Clostridium difficile, Clostridium perfringens* und *Staphylococcus aureus.*

11. Verwendung nach Anspruch 10, wobei das hergestellte Medikament für die Behandlung der Darmerkrankung vorgesehen ist.

12. Verwendung nach Anspruch 10, wobei das hergestellte Medikament zur Verhütung der Darmerkrankung vorgesehen ist.

13. Verwendung nach Anspruch 10, wobei das hergestellte Medikament auch dafür vorgesehen ist, die Konzentration von vancomycinresistenten Enterokokken im Darm nicht zu erhöhen.

14. Verwendung nach Anspruch 10, wobei das hergestellte Medikament auch dafür vorgesehen ist, die Konzentration von vancomycinresistenten Enterokokken im Darm zu verringern.

## Revendications

1. Composé de formule I 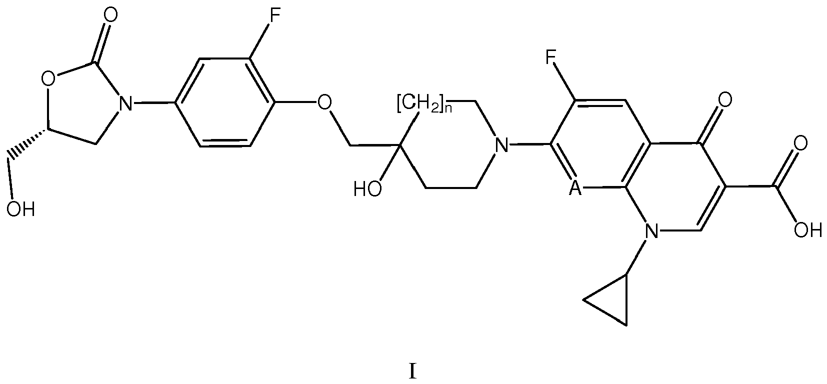 où
A représente N ou CH ; et
n représente 0 ou 1 ;
ou sel pharmaceutiquement acceptable de celui-ci, pour une utilisation dans la prévention ou le traitement de maladies intestinales causées par une bactérie sélectionnée parmi *Clostridium difficile, Clostridium perfringens* ou *Staphylococcus aureus.*

2. Composé de formule I tel que défini à la revendication 1, ou sel pharmaceutiquement acceptable de celui-ci, pour l'utilisation mentionnée à la revendication 1, où A représente CH.

3. Composé de formule I tel que défini à la revendication 1 ou 2, ou sel pharmaceutiquement acceptable de celui-ci, pour l'utilisation mentionnée à la revendication 1, où n représente 1.

4. Composé de formule I tel que défini à la revendication 1, qui est sélectionné parmi les suivants :
- acide 1-cyclopropyl-6-fluoro-7-{4-[2-fluoro-4-((*R*)-5-hydroxyméthyl-2-oxo-oxazolidin-3-yl)-phénoxyméthyl]-4-hydroxy-pipéridin- 1-yl} -4-oxo-1,4-dihydro-quinoléine-3-carboxylique ;
- acide 1-cyclopropyl-6-fluoro-7-{4-[2-fluoro-4-((*R*)-5-hydroxyméthyl-2-oxo-oxazolidin-3-yl)-phénoxyméthyll-4-hydroxy-pipéridin-1-yl}-4-oxo-1,4-dihydro-[1,8]naphtyridine-3-carboxylique ; et
- acide 1-cyclopropyl-6-fluoro-7-{3-[2-fluoro-4-((*R*)-5-hydroxyméthyl-2-oxo-oxazolidin-3-yl)-phénoxyméthyl]-3-hydroxy-pyrrolidin-1-yl } -4-oxo-1,4-dihydro-quinoléine-3-carboxylique ;
ou sel pharmaceutiquement acceptable d'un tel composé, pour une utilisation dans la prévention ou le traitement de maladies intestinales causées par une bactérie sélectionnée parmi *Clostridium difficile, Clostridium perfringens* ou *Staphylococcus aureus.*

5. Composé de formule I tel que défini à la revendication 1 ou sel pharmaceutiquement acceptable de celui-ci, qui correspond à l'acide 1-cyclopropyl-6-fluoro-7-{4-[2-fluoro-4-((*R*)-5-hydroxyméthyl-2-oxo-oxazolidin-3-yl)-phénoxyméthyl]-4-hydroxy-pipéridin-1-yl}-4-oxo-1,4-dihydro-quinoléine-3-carboxylique ou à un sel pharmaceutiquement acceptable de celui-ci, pour la prévention ou le traitement de maladies intestinales causées par une bactérie sélectionnée parmi *Clostridium difficile, Clostridium perfringens* ou *Staphylococcus aureus.*

6. Composé de formule I tel que défini à l'une des revendications 1 à 5, ou sel pharmaceutiquement acceptable de celui-ci, pour la prévention ou le traitement de maladies intestinales causées par *Clostridium difficile.*

7. Composé de formule I tel que défini à l'une des revendications 1 à 5, ou sel pharmaceutiquement acceptable de celui-ci, pour la prévention ou le traitement de maladies intestinales causées par une souche de *Clostridium difficile* productrice de toxines.

8. Composé de formule I tel que défini à l'une des revendications 1 à 5, ou sel pharmaceutiquement acceptable de celui-ci, pour la prévention ou le traitement des maladies diarrhéiques associées à des souches entérotoxicogènes de *Clostridium difficile, Clostridium perfringens* ou *Staphylococcus aureus* sans augmentation de la concentration en entérocoques résistants à la vancomycine dans l'intestin.

9. Composé de formule I tel que défini à l'une des revendications 1 à 5 ou sel pharmaceutiquement acceptable de celui-ci, pour la prévention ou le traitement des maladies diarrhéiques associées à des souches entérotoxicogènes de *Clostridium difficile, Clostridium perfringens* ou *Staphylococcus aureus* et la réduction de la concentration en entérocoques résistants à la vancomycine dans l'intestin.

10. Utilisation d'un composé de formule I tel que défini à l'une des revendications 1 à 5 ou d'un sel pharmaceutiquement acceptable d'un tel composé dans la fabrication d'un médicament conçu pour prévenir ou traiter une maladie intestinale causée par une bactérie sélectionnée parmi *Clostridium difficile, Clostridium perfringens* et *Staphylococcus aureus.*

11. Utilisation selon la revendication 10, où le médicament fabriqué est conçu pour traiter la maladie intestinale.

12. Utilisation selon la revendication 10, où le médicament fabriqué est conçu pour prévenir la maladie intestinale.

13. Utilisation selon la revendication 10, où le médicament fabriqué est également conçu pour ne pas augmenter la concentration en entérocoques résistants à la vancomycine dans l'intestin.

14. Utilisation selon la revendication 10, où le médicament fabriqué est également conçu pour diminuer la concentration en entérocoques résistants à la vancomycine dans l'intestin.
